Europäisches Patentamt

European Patent Office

Office européen des brevets

(19)

(11) Veröffentlichungsnummer: **0 518 881 B1**

## (12) EUROPÄISCHE PATENTSCHRIFT

(45) Veröffentlichungstag der Patentschrift: **28.12.94**

(51) Int. Cl.5: **C07H 15/04**

(21) Anmeldenummer: **91904159.0**

(22) Anmeldetag: **26.02.91**

(86) Internationale Anmeldenummer:
**PCT/EP91/00353**

(87) Internationale Veröffentlichungsnummer:
**WO 91/13896 (19.09.91 91/22)**

(54) **VERFAHREN ZUR SULFIERUNG ALKYLGLYKOSIDHALTIGER GEMISCHE.**

(30) Priorität: **05.03.90 DE 4006841**

(43) Veröffentlichungstag der Anmeldung:
**23.12.92 Patentblatt 92/52**

(45) Bekanntmachung des Hinweises auf die
Patenterteilung:
**28.12.94 Patentblatt 94/52**

(84) Benannte Vertragsstaaten:
**DE ES FR GB IT**

(56) Entgegenhaltungen:
**EP-A- 0 186 242**
**EP-A- 0 363 601**
**WO-A-88/01640**

(73) Patentinhaber: **Henkel Kommanditgesellschaft auf Aktien**

**D-40191 Düsseldorf (DE)**

(72) Erfinder: **FABRY, Bernd**
**Danziger Strasse 31**
**D-4052 Korschenbroich 1 (DE)**
Erfinder: **WUTHEN, Manfred**
**Hermann-Löns-Weg 102**
**D-5650 Solingen 11 (DE)**
Erfinder: **SCHUMACHER, Astrid**
**Gleiwitzerstrasse 6**
**D-4000 Düsseldorf 1 (DE)**

EP 0 518 881 B1

**Beschreibung**

Die Erfindung betrifft ein Verfahren zur Sulfierung alkylglykosidhaltiger Gemische.

Die Sulfierung von Alkylglykosiden führt zu anionischen Tensiden mit Sulfatstruktur, die sich durch günstige Schaum-, Wasch- und Reinigungseigenschaften sowie eine hohe ökotoxikologische Verträglichkeit auszeichnen. Infolge des hohen Schmelzpunktes der Alkylglykoside kann die Sulfierung jedoch nicht in technisch üblicher Weise, d. h. in der Schmelze durchgeführt werden. Es ist vielmehr erforderlich, zunächst eine Lösung des Alkylglykosids in einem inerten organischen Lösungsmittel herzustellen, ehe die Umsetzung mit dem Sulfieragens stattfinden kann. So beschreibt z. B. die europäische Patentanmeldung EP-A-0 186 242 ein Verfahren zur Sulfierung von Alkylglykosiden mit gasförmigem Schwefeltrioxid in einer Mischung aus Dimethylformamid und Dichlorethan. Gemäß der Lehre der internationalen Patentanmeldung WO 88/1640 kann die Sulfierung von Alkylglykosiden in Dimethylformamid auch mit einem Komplex aus Schwefeltrioxid und einem Amin durchgeführt werden.

Bei den genannten Verfahren muß nach Sulfierung und Neutralisation die organische Lösungsmittelkomponente abgetrennt werden. Dieser Schritt ist mit einem hohen apparativen und zeitlichen Aufwand verbunden; in vielen Fällen bleiben auch Spuren des Lösungsmittels im Produkt zurück, was für manche Anwendungen unerwünscht ist.

Das zu lösende technische Problem bestand nun darin, ein Verfahren zur Sulfierung von Alkylglykosiden zu entwickeln, das ohne Lösungsmittelabtrennung auskommt.

Gegenstand der Erfindung ist ein Verfahren zur Sulfierung alkylglykosidhaltiger Gemische, bei dem man ein bei Temperaturen zwischen 25 und 80°C flüssiges, homogenes Gemisch aus

a) einem Alkylglykosid der allgemeinen Formel (I),

$$R\text{-}O\text{-}(G)_p, \qquad (I)$$

wobei G ein Symbol für eine Glycose-Einheit darstellt, die sich von einem Zucker mit 5 oder 6 Kohlenstoffatomen ableitet, der Oligomerisierungsgrad p für eine Zahl zwischen 1 und 10 und R für einen Alkylrest mit 6 bis 22 Kohlenstoffatomen steht und

b) einer Verbindung ausgewählt aus der Gruppe gebildet aus

$b^1$) Fettalkoholen mit 6 bis 22 Kohlenstoffatomen,

$b^2$) Anlagerungsprodukten von durchschnittlich 0,1 bis 10 Mol Ethylenoxid an Fettalkohole mit 6 bis 22 Kohlenstoffatomen,

$b^3$) gesättigten und ungesättigten Fettsäuren mit 6 bis 22 Kohlenstoffatomen und

$b^4$) Estern gesättigter und ungesättigter Fettsäuren mit 6 bis 22 Kohlenstoffatomen mit Alkoholen mit 1 bis 5 Kohlenstoffatomen

sulfiert und anschließend mit wässrigen Basen neutralisiert.

Bei der Mischungskomponente a) handelt es sich um solche Alkylglykoside, bei denen sich die Glycose-Einheit G in der allgemeinen Formel (I) von Aldosen bzw. Ketosen ableitet. Vorzugsweise werden wegen der besseren Reaktionsfähigkeit die reduzierend wirkenden Saccharide, die Aldosen, verwendet. Unter den Aldosen kommt wegen ihrer leichten Zugänglichkeit und technischen Verfügbarkeit insbesondere die Glucose in Betracht. Die als Ausgangsstoffe besonders bevorzugt eingesetzten Alkylglykoside sind daher die Alkylglucoside.

Die Indexzahl p in der allgemeinen Formel (I) gibt den Oligomerisierungsgrad, d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkylglykosid eine analytische ermittelte rechnerische Größe, die meistens eine gebrochene Zahl darstellt. Vorzugsweise werden Alkylglykoside mit einem mittleren Oligomerisierungsgrad p von 1,1 bis 3,0 verwendet. Besonders bevorzugt sind solche Alkylglykoside, deren Oligomerisierungsgrad kleiner als 1,5 ist und insbesondere zwischen 1,1 und 1,4 liegt.

Der Alkylrest R leitet sich von primären Alkoholen mit 6 bis 22, vorzugsweise 12 bis 18 Kohlenstoffatomen ab. Typische Beispiele sind Capronalkohol, Caprylalkohol, Caprinalkohol, Laurylalkohol, Myristylalkohol, Cetylalkohol, Stearylalkohol und Behenylalkohol sowie technische Fraktionen, die neben den genannten gesättigten Alkoholen auch Anteile an ungesättigten Alkoholen enthalten können und die auf Basis von natürlichen Fetten und Ölen, beispielsweise Palmöl, Palmkernöl, Kokosöl oder Rindertalg gewonnen werden. Der Einsatz von technischem Kokosalkohol ist hierbei besonders bevorzugt.

Neben den genannten Fettalkoholen können sich die Alkylglykoside auch von synthetischen primären Alkoholen mit 6 bis 22 Kohlenstoffatomen, insbesondere den sogenannten Oxoalkoholen ableiten, die einen Anteil von 5 bis 40 Gew.-% verzweigter Isomeren aufweisen.

2

Verfahren zur Herstellung dieser Alkylglykoside sind beispielsweise in den amerikanischen Patentschriften US 3,547,828 und US 3,839,318 sowie der deutschen Patentanmeldung DE-A-37 23 826 beschrieben.

Als Mischungskomponente b) kommen Fettalkohole in Betracht, bei denen es sich um primäre Alkohole mit 6 bis 22, insbesondere 12 bis 18 Kohlenstoffatomen handelt. Typische Beispiele hierfür sind Capronalkohol, Caprylalkohol, Caprinalkohol, Laurylalkohol, Myristylalkohol, Cetylalkohol, Stearylalkohol und Behenylalkohol. Wie in der Fettchemie üblich, können die Fettalkohole auch als technische Gemische vorliegen, die neben den genannten gesättigten Alkoholen auch Anteile an ungesättigten Alkoholen enthalten können und die auf Basis von natürlichen Fetten und Ölen, beispielsweise Palmöl, Palmkernöl, Kokosöl oder Rindertalg gewonnen werden. Anionische Tensidgemische mit besonders vorteilhaften Eigenschaften werden erhalten, wenn man als Mischungskomponente b) von technischem Kokosalkohol ausgeht.

Neben den genannten Fettalkoholen können auch synthetische primäre Alkohole mit 6 bis 22 Kohlenstoffatomen, insbesondere sogenannte Oxoalkohole zum Einsatz gelangen, die einen Anteil von 20 bis 40 Gew.-% verzweigter Isomeren aufweisen.

Die primären Alkohole können gegebenenfalls auch als Anlagerungsprodukte von Ethylenoxid vorliegen. Zum Einsatz gelangen dabei Addukte von durchschnittlich 0,1 bis 10, vorzugsweise 1 bis 8 Mol Ethylenoxid an die genannten Fettalkohole, die in Gegenwart typischer Alkoxylierungskatalysatoren, beispielsweise Natriummethylat oder Kaliumhydroxid bei einem Druck von 4 bis 5 bar und Temperaturen von 140 bis 220 °C erhalten werden. Anionische Tensidgemische mit besonders vorteilhaften Eigenschaften werden erhalten, wenn man als Mischungskomponente b) von dem Hydrotalcit-katalysierten Anlagerungsprodukt von durchschnittlich 5 Mol Ethylenoxid an einen technischen $C_{12/14}$-Kokosalkoholschnitt ausgeht, das einen freien Fettalkoholanteil von weniger als 1 Gew.-% aufweist.

Fettalkohole und deren Anlagerungsprodukte von Ethylenoxid, die als Mischungskomponente b) dienen, werden während der Sulfierung sulfatiert.

Als Mischungskomponente b) kommen weiter gesättigte und ungesättigte Fettsäuren in Betracht, die 6 bis 22, vorzugsweise 12 bis 20 Kohlenstoffatome und 0, 1, 2 oder 3, vorzugsweise jedoch eine Doppelbindung aufweisen. Typische Beispiele hierfür sind Capronsäure, Caprylsäure, Caprinsäure, Laurinsäure, Myristinsäure, Palmitoleinsäure, Palmitinsäure, Ölsäure, Elaidinsäure, Petroselinsäure, Linolsäure, Linolensäure, Erucasäure und Behensäure. Wie in der Fettchemie üblich können diese Säuren auch als technische Gemische vorliegen, wie sie auf Basis von natürlichen Fetten und Ölen, beispielsweise Palmöl, Palmkernöl, Kokosöl, Sonnenblumenöl, Rüböl oder Rindertalg gewonnen werden. Die Verwendung von technischer Ölsäure mit einem Anteil von weniger als 10 Gew.-% Nebenbestandteile ist hierbei bevorzugt.

Die Fettsäuren können auch in Form von Estern mit aliphatischen Alkoholen mit 1 bis 5 Kohlenstoffatomen vorliegen. Bevorzugt sind hierbei die Methylester technischer Kokos- und Talgfettsäuren.

Fettsäuren und deren Ester, die als Mischungskomponente b) dienen, werden während der Sulfierung sulfoniert.

Bei der Auswahl der Mischungskomponente b) sind generell Fettalkohole bevorzugt, da Gemische enthaltend Alkylglykoside und Fettalkohole im Gewichtsverhältnis 20 : 80 bis 30 : 70 bei der technischen Herstellung von Alkylglykosiden anfallen und somit unmittelbar oder nach Abtrennung eines Teils der Alkoholkoponente in die Sulfierung eingesetzt werden können; ein Mischen der reinen Komponenten ist in diesem Fall nicht erforderlich.

In allen anderen Fällen müssen die Komponenten a) und b) auf mechanischem Wege vermischt werden. Dies erfolgt vorteilhafterweise durch Zusammengeben der Komponenten bei Temperaturen zwischen 25 und 80 °C, wobei durch intensives Rühren sichergestellt wird, daß die Mischung ein hohes Maß an Homogenität aufweist. Das Mischungsverhältnis a) : b) kann 60 : 40 bis 5 : 95, insbesondere 50 : 50 bis 15 : 85 Gew.-% betragen. Die Sulfierung der Ausgangsgemische aus a) und b) kann nach gängigen Verfahren, beispielsweise mit Schwefelsäure, Oleum, Chlorsulfonsäure oder Amidosulfonsäure durchgeführt werden. Vorzugsweise erfolgt sie jedoch mit gasförmigem Schwefeltrioxid in der für Fettsäureniedrigalkylester bekannten Weise [J.Falbe (ed.), "Surfactants in consumer products", Springer Verlag, Berlin-Heidelberg, 1987, S.61], wobei Reaktoren, die nach dem Fallfilmprinzip arbeiten, besonders bevorzugt sind. Dabei wird das Schwefeltrioxid mit einem inerten Gas, vorzugsweise Luft oder Stickstoff verdünnt und in Form eines Gasgemisches, welches das $SO_3$ in einer Konzentration von 1 bis 8, insbesondere 2 bis 5 Vol.-% enthält, eingesetzt.

Das Einsatzverhältnis des gasförmigem Schwefeltrioxids beträgt 0,9 bis 1,6 Mol, vorzugsweise jedoch 1,0 bis 1,3 Mol Schwefeltrioxid pro Mol Ausgangsmischung.

Die Sulfierreaktion wird bei Temperaturen von 25 bis 80 °C, vorzugsweise 30 bis 75 °C durchgeführt. Eine genaue Temperaturkontrolle ist dabei für die Durchführung der Reaktion von äußerster Wichtigkeit: bei einer zu niedrigen Reaktionstemperatur zeigt die saure Sulfiermischung kein ausreichendes Fließvermögen, während zu hohe Temperaturen eine Verkohlung oder Karamelisierung des Produktes zur Folge haben.

Multipliziert man die durchschnittliche Anzahl der Kohlenstoffatome der Mischungskomponente b) mit dem Faktor 3,3 °C, erhält man die niedrigst mögliche ($T_{min}$), mit dem Faktor 5 °C, hingegen die höchst mögliche Sulfiertemperatur ($T_{max}$) beliebiger Gemische aus a) und b) in den angegebenen Mischungsgrenzen. Für jede in der Komponente b) enthaltene Ethylenoxideinheit erhöhen sich $T_{min}$ und $T_{max}$ um 2 °C, während jede Doppelbindung in Komponente b) beide Grenzwerte um den Faktor 2 erniedrigt.

Die bei der Sulfierung anfallenden sauren Sulfierprodukte werden in wässrige Basen eingerührt, neutralisiert und auf einen pH-Wert von 7,5 bis 9,0 eingestellt. Für die Neutralisation kommen Basen ausgewählt aus der Gruppe gebildet von Alkylimetallhydroxiden wie Natrium, Kalium- und Lithiumhydroxid, Erdalkalimetalloxiden und -hydroxiden wie Magnesiumoxid, Calciumoxid und Calciumhydroxid, Ammoniak, Mono-, Di- und Tri-$C_{2-4}$-Alkanolaminen, beispielsweise Mono-, Di- und Triethanolamin sowie primären, sekundären oder tertiären $C_{1-4}$-Alkylaminen in Betracht. Die Neutralisationsbasen gelangen dabei vorzugsweise in Form mehr oder minder konzentrierter wässriger Lösungen zum Einsatz, wobei 25 bis 55 gew.-%ige Natriumhydroxidlösungen bevorzugt sind.

Das Sulfierprodukt stellt in Abhängigkeit der Mischungskomponente b) ein komplexes Gemisch dar, das neben primären Alkylglucosidsulfaten Sulfierprodukte der Komponente b) enthält, bei denen es sich um Verbindungen aus der Gruppe der Alkylsulfate, Alkylethersulfate, alpha- oder innenständigen Sulfofettsäuren und deren Estern handelt. Als Nebenprodukte finden sich geringe Anteile Glucose und Oligoglucose.

Die nach dem erfindungsgemäßen Verfahren erhältlichen anionischen Tensidgemische können nach Neutralisation in an sich bekannter Weise durch Zusatz von Wasserstoffperoxid- oder Natriumhypochloritlösung gebleicht werden. Dabei werden, bezogen auf den Feststoffgehalt in der Lösung der Sulfierprodukte, 0,2 bis 2 Gew.-% Wasserstoffperoxid, berechnet als 100 gew.-%ige Substanz, oder entsprechende Mengen Natriumhypochlorit eingesetzt. Der pH-Wert der Lösungen kann unter Verwendung geeigneter Puffermittel, z. B. mit Natriumphosphat oder Citronensäure konstant gehalten werden. Zur Stabilisierung gegen Bakterienbefall empfiehlt sich ferner eine Konservierung, z. B. mit Formaldehydlösung, p-Hydroxybenzoat, Sorbinsäure oder anderen bekannten Konservierungsstoffen.

Die nach dem erfindungsgemäßen Verfahren erhältlichen anionischen Tensidgemische finden Verwendung als oberflächenaktive Mittel, so z. B. zur Herstellung von flüssigen Waschmitteln oder Reinigungsmitteln für harte Oberflächen.

Die nachfolgenden Beispiele sollen den Gegenstand der Erfindung näher erläutern.

## Beispiele

Herstellung der Ausgangsprodukte

**Herstellung Von $C_{12/14}$-Kokosalkylglucosid (a1).** In einem 2-l-Dreihalskolben mit Rührer und Rückflußkühler wurden 873 g (4,5 Mol) eines technischen $C_{12/14}$-Kokosalkoholschnittes (Lorol Spezial[R], Hydroxylzahl = 289, Handelsprodukt der Fa.Henkel) vorgelegt und in Gegenwart von 5,5 Mol p-Toluolsulfonsäure mit 180 g (1 Mol) wasserfreier Glucose versetzt. Die Reaktionsmischung wurde ca. 5 h bei 130 bis 150 °C gehalten und gebildetes Kondensationswasser im Vakuum abgezogen. Nach Neutralisation mit Magnesiumoxid und Abtrennung des überschüssigen Fettalkohols wurde das Kokosalkylglucosid (Hydroxylzahl = 361) in praktisch quantitativer Ausbeute erhalten. Der Oligomerisierungsgrad p des Produktes betrug 1,3.

**Herstellung von Octylglucosid (a2).** Vorschrift (a1) wurde unter Einsatz von 594 g (4,5 Mol) Octanol (Lorol C8[R], Hydroxylzahl = 425, Handelsprodukt der Fa.Henkel) wiederholt. Das in praktisch quantitativer Ausbeute gewonnene Octylglucosid wies eine Hydroxylzahl von 146 auf. Der Oligomerisierungsgrad p des Produktes betrug 1,3.

**Herstellung von $C_{12/18}$-Kokosalkylglucosid (a3).** Vorschrift (a1) wurde unter Einsatz von 935 g (4,5 Mol) $C_{12/18}$-Kokosfettalkohol (Lorol Technisch[R], Hydroxylzahl = 270, Handelsprodukt der Fa. Henkel) wiederholt. Das in praktisch quantitativer Ausbeute gewonnene $C_{12/18}$-Kokosalkylglucosid wies eine Hydroxylzahl von 89 auf. Der Oligomerisierungsgrad p des Produktes betrug 1,3.

Beispiele 1.1 - 1.13:

**Allgemeine Arbeitsvorschrift Verfahren A:** In einem 500 ml Becherglas wurden bei 30 bis 60 °C unter Rühren 0,2 Mol eines Alkylglucosids gemäß a1, a2 oder a3 in 0,8 Mol der Mischungskomponente b) ausgewählt aus der Gruppe gebildet von Fettalkoholen, deren Ethylenoxidanlagerungsprodukten sowie gesättigten und ungesättigten Fettsäuren und deren Niedrigalkylestern, eingetragen.

1 Mol der Mischung wurde in einen 0,5-l-Sulfierreaktor mit Mantelkühlung überführt und bei 30 bis 75 °C mit 1 bis 1,3 Mol gasförmigem Schwefeltrioxid umgesetzt. Das $SO_3$ wurde durch Erhitzen einer entsprechenden Menge 65 gew.-%igen Oleums ausgetrieben, mit Stickstoff auf eine Konzentration von 5 Vol.-% verdünnt und innerhalb von 33 bis 40 min in die Mischung eingeleitet. Nach der Sulfierung wurde das saure Reaktionsgemisch in wässrige 25 gew.-%ige Natriumhydroxidlösung eingetragen, neutralisiert und auf pH = 8 eingestellt. Die Reaktionsbedingungen und Kenndaten der Produkte sind in Tab.1 zusammengefaßt.

Beispiele 2.1 - 2.2

**Allgemeine Arbeitsvorschrift Verfahren B:** Verfahren A wurde unter Einsatz einer Mischung aus 0,5 Mol eines Alkylglucosids a1, a2 oder a3 und einer Mischungskomponente b) ausgewählt aus der Gruppe gebildet von Fettalkoholen und deren Anlagerungsprodukten von Ethylenoxid, gesättigten und ungesättigten Fettsäuren und deren Niedrigalkylestern wiederholt. Die Reaktionsbedingungen und Kenndaten der Produkte sind in Tab.1 zusammengefaßt.

Beispiele 3.1 - 3.2:

**Allgemeine Arbeitsvorschrift Verfahren C:** In einem kontinuierlich arbeitenden Fallfilmreaktor (Länge 120 cm, Querschnitt 1 cm, Eduktdurchsatz 600 g/h) mit Mantelkühlung und seitlicher $SO_3$-Begasung wurden 2440 g (10 Mol) der Mischung bestehend aus $C_{12/14}$-Kokosfettalkohol und $C_{12/14}$-Kokosalkylglucosid gemäß Beispiel 1.1 mit 890 g (10 Mol) Schwefeltrioxid zur Reaktion gebracht. Die Aufarbeitung der sauren Reaktionsprodukte erfolgte gemäß Verfahren A. Die Reaktionsbedingungen und Kenndaten der Produkte sind in Tab.1 zusammengefaßt.

Beispiel 5.1 - 5.2:

**Allgemeine Arbeitsvorschrift Verfahren D:** Verfahren C wurde unter Einsatz eines technischen Gemisches bestehend aus 80 Gew.-% $C_{12/14}$-Kokosfettalkohol und 20 Gew.-% $C_{12/14}$-Kokosalkylglucosid wiederholt, wie es bei der technischen Herstellung von Alkylglucosiden als Zwischenstufe anfällt. Die Reaktionsbedingungen und Kenndaten der Produkte sind in Tab.1 zusammengefaßt.

## Tab.1: Reaktionsbedingungen und Kenndaten der Produkte
Prozentangaben als Gew.-%

| Bsp. | V. | Komp a | Komp b | Komp a g | Komp b g | $SO_3$ Mol | T °C | M$^{WAS}$ | WAS % | US % | $Na_2SO_4$ % | $H_2O$ % |
|---|---|---|---|---|---|---|---|---|---|---|---|---|
| 1.1 | A | a1 | b1 | 89 | 155 | 1.0 | 40-55 | 346 | 14.2 | 0.6 | 1.6 | 83.6 |
| 1.2 | A | a1 | b1 | 89 | 155 | 1.3 | 40-55 | 377 | 14.9 | 0.5 | 1.5 | 83.1 |
| 1.3 | A | a1 | b2 | 89 | 106 | 1.0 | 32-38 | 297 | 12.6 | 0.7 | 0.5 | 86.2 |
| 1.4 | A | a1 | b3 | 89 | 166 | 1.0 | 50-60 | 357 | 10.4 | 1.9 | 0.4 | 87.3 |
| 1.5 | A. | a1 | b4 | 89 | 206 | 1.0 | 60-70 | 397 | 9.9 | 9.9 | 0.4 | 87.6 |
| 1.6 | A | a1 | b5 | 89 | 331 | 1.0 | 40-45 | 522 | 11.5 | 1.5 | 0.6 | 86.4 |
| 1.7 | A* | a1 | b6 | 89 | 225 | 1.0 | 30-40 | 416 | 12.6 | 1.2 | 1.1 | 85.1 |
| 1.8 | A# | a1 | b7 | 89 | 191 | 1.0 | 40-60 | 387 | 8.5 | 8.1 | 0.9 | 82.5 |
| 1.9 | A# | a1 | b8 | 89 | 241 | 1.0 | 70-72 | 432 | 6.4 | 9.8 | 2.4 | 81.4 |
| 1.10 | A | a2 | b2 | 68 | 106 | 1.0 | 30-35 | 276 | 12.6 | 0.8 | 0.5 | 86.1 |
| 1.11 | A | a2 | b1 | 68 | 155 | 1.0 | 40-45 | 325 | 14.1 | 0.7 | 1.3 | 83.9 |
| 1.12 | A | a3 | b3 | 92 | 166 | 1.0 | 60-75 | 360 | 10.3 | 1.9 | 0.6 | 87.2 |
| 1.13 | A | a3 | b1 | 92 | 155 | 1.0 | 60-75 | 349 | 11.5 | 1.8 | 0.6 | 86.1 |
| 2.1 | B | a1 | b1 | 222 | 97 | 1.0 | 40-55 | 421 | 13.9 | 0.5 | 1.5 | 84.1 |
| 2.2 | B | a1 | b1 | 222 | 97 | 1.3 | 40-55 | 452 | 14.4 | 0.4 | 1.3 | 83.9 |
| 3.1 | C | a1 | b1 | 89 | 155 | 1.0 | 40-55 | 346 | 14.1 | 0.6 | 1.6 | 83.7 |
| 3.2 | C | a1 | b1 | 89 | 155 | 1.3 | 40-55 | 377 | 15.0 | 0.5 | 1.4 | 83.1 |
| 4.1 | D | a | b1 | 89 | 155 | 1.0 | 40-55 | 346 | 14.3 | 0.5 | 1.5 | 83.7 |
| 4.2 | D | a | b1 | 89 | 155 | 1.3 | 40-55 | 377 | 14.9 | 0.5 | 1.4 | 83.2 |

Legende: V       = Verfahren

Komp.a   = Komponente a), Alkylglucosid

Komp.b   = Komponente b)

$SO_3$       = Menge Mol $SO_3$/ Mol Einsatzstoffe

M$^{WAS}$    = Mittleres Molgewicht der anionischen Tensidmischung

WAS      = Aniontensidgehalt

US       = Unsulfierte Anteile

OHZ      = Hydroxylzahl

VZ       = Verseifungszahl


a1       = $C_{12/14}$-Kokosalkylglucosid

a2       = Octylglucosid

a3       = $C_{12/18}$-Kokosalkylglucosid


b1       = $C_{12/14}$-Kokosfettalkohol, OHZ = 289

b2       = Octanol

b3       = $C_{12/18}$-Kokosfettalkohol, OHZ = 270

b4       = $C_{16/18}$-Talgfettalkohol, OHZ 217

b5       = $C_{12/14}$-Kokosfettalkyl-5 Mol EO-ether mit eingeengter Homologenverteilung, OHZ = 135

b6       = Ölsäure, technisch (Reinheit: > 90 Gew.-%)

b7       = $C_{12/14}$-Kokosfettsäuremethylester, VZ = 235

b8       = $C_{16/18}$-Talgfettsäuremethylester, VZ = 186


* Abweichend von Verfahren A wurde das Produkt vor der Neutralisation noch 2 h bei 95°C erhitzt.

\# Abweichend von Verfahren A wurde das saure Sulfierprodukt vor der Neutralisation noch 15 min bei 80°C gealtert.


Der Aniontensidgehalt (WAS) und die Unsulfierten Anteile(US) wurden nach den DGF-Einheitsmethoden, Stuttgart 1950-1984, H-III-10 bzw. G-II-6b ermittelt.

**Patentansprüche**

1. Verfahren zur Sulfierung alkylglykosidhaltiger Gemische, dadurch gekennzeichnet, daß man ein bei Temperaturen zwischen 25 und 80°C flüssiges, homogenes Gemisch aus

    a) einem Alkylglykosid der allgemeinen Formel **(I)**,

    **R-O-(G)$_p$**     **(I)**

    wobei G ein Symbol für eine Glycose-Einheit darstellt, die sich von einem Zucker mit 5 oder 6 Kohlenstoffatomen ableitet, der Oligomerisierungsgrad p für eine Zahl zwischen 1 und 10 und R für einen Alkylrest mit 6 bis 22 Kohlenstoffatomen steht und
    b) einer Verbindung ausgewählt aus der Gruppe gebildet aus
        b[1])     Fettalkoholen mit 6 bis 22 Kohlenstoffatomen,
        b[2])     Anlagerungsprodukten von durchschnittlich 0,1 bis 10 Mol Ethylenoxid an Fettalkohole mit 6 bis 22 Kohlenstoffatomen,
        b[3])     gesättigten und ungesättigten Fettsäuren mit 6 bis 22 Kohlenstoffatomen und
        b[4])     Estern gesättigter und ungesättigter Fettsäuren mit 6 bis 22 Kohlenstoffatomen mit Alkoholen mit 1 bis 5 Kohlenstoffatomen
    sulfiert und anschließend mit wässrigen Basen neutralisiert.

2. Verfahren nach Anspruch 1, dadurch gekennzeichnet, daß Alkylglucoside verwendet werden, bei denen sich die Glycoseeinheit G in der allgemeinen Formel **(I)** von Aldosen bzw. Ketosen, insbesondere der Glucose ableitet, der mittlere Oligomerisierungsgrad p eine Zahl von 1,1 bis 3,0, insbesondere 1,1 bis 1,4, und R einen Alkylrest mit 12 bis 18 Kohlenstoffatomen darstellt.

3. Verfahren nach einem der Ansprüche 1 und 2, dadurch gekennzeichnet, daß als Mischungskomponente b) Fettalkohole mit 12 bis 18 Kohlenstoffatomen verwendet werden.

4. Verfahren nach Anspruch 3, dadurch gekennzeichnet, daß als Mischungskomponente b) ein technischer Kokosalkohol verwendet wird.

5. Verfahren nach einem der Ansprüche 1 und 2, dadurch gekennzeichnet, daß als Mischungskomponente b) Anlagerungsprodukte von durchschnittlich 1 bis 8 Mol Ethylenoxid an Fettalkohole mit 6 bis 22 Kohlenstoffatomen verwendet werden.

6. Verfahren nach Anspruch 5, dadurch gekennzeichnet, daß als Mischungskomponente b) das Hydrotalcit-katalysierte Anlagerungsprodukt von durchschnittlich 5 Mol Ethylenoxid an einen technischen C$_{12/14}$-Kokosalkoholschnitt verwendet wird.

7. Verfahren nach einem der Ansprüche 1 und 2, dadurch gekennzeichnet, daß als Mischungskomponente b) gesättigte oder ungesättigte Fettsäuren mit 12 bis 20 Kohlenstoffatomen und 0, 1, 2 oder 3 Doppelbindungen verwendet werden.

8. Verfahren nach Anspruch 7, dadurch gekennzeichnet, daß als Mischungskomponente b) technische Ölsäure mit einem Anteil von weniger als 10 Gew.-% gesättigter Nebenprodukte verwendet wird.

9. Verfahren nach einem der Ansprüche 1 und 2, dadurch gekennzeichnet, daß als Mischungskomponente b) Ester von Fettsäuren mit 12 bis 22 Kohlenstoffatomen und 0, 1 2 oder 3 Doppelbindungen mit aliphatischen Alkoholen mit 1 bis 5 Kohlenstoffatomen verwendet werden.

10. Verfahren nach Anspruch 9, dadurch gekennzeichnet, daß als Mischungskomponente b) Methylester technischer Kokos- und Talgfettsäuren verwendet werden.

11. Verfahren nach mindestens einem der Ansprüche 1 bis 10, dadurch gekennzeichnet, daß Mischungen verwendet werden, bei denen das Mischungsverhältnis von Komponente a) zu Komponente b) 60 : 40 bis 5 : 95 Gew.-% beträgt.

EP 0 518 881 B1

**12.** Verfahren nach Anspruch 11, dadurch gekennzeichnet, daß Mischungen verwendet werden, bei denen das Mischungsverhältnis von Komponente a) zu Komponente b) 50 : 50 bis 15 : 85 Gew.-% beträgt.

**13.** Verfahren nach mindestens einem der Ansprüche 1 bis 12, dadurch gekennzeichnet daß Mischungen von $C_{12/14}$-Kokosalkylglucosiden und einem technischen $C_{12/14}$-Kokosfettalkoholschnitt im Gewichtsverhältnis 20 : 80 bis 30 : 70 verwendet werden.

**14.** Verfahren nach mindestens einem der Ansprüche 1 bis 13, dadurch gekennzeichnet, daß die Sulfierung der Gemische aus a) und b) mit Schwefelsäure, Oleum, Chlorsulfonsäure oder Amidosulfonsäure, insbesondere aber gasförmigem Schwefeltrioxid im Gemisch mit einem Inertgas durchgeführt wird.

**15.** Verfahren nach mindestens einem der Ansprüche 1 bis 14, dadurch gekennzeichnet, daß die Sulfierung mit einem molaren Einsatzverhältnis von Ausgangsmischung zu gasförmigem Schwefeltrioxid von 1: 0,9 bis 1 : 1,6 durchgeführt wird.

**16.** Verfahren nach mindestens einem der Ansprüche 1 bis 15, dadurch gekennzeichnet, daß die Sulfierung bei Temperaturen von 25 bis 80 °C durchgeführt wird.

**17.** Verfahren nach mindestens einem der Ansprüche 1 bis 16, dadurch gekennzeichnet, daß die Neutralisation der sauren Sulfierprodukte mit wässrigen Basen ausgewählt aus der Gruppe der Alkalimetallhydroxide, Erdalkalimetalloxide und -hydroxide, Ammoniak, Mono-, Di- und Tri-$C_{2-4}$-Alkanolamine sowie primären, sekundären und tertiären $C_{1-4}$-Alkylamine durchgeführt wird.

**Claims**

**1.** A process for the sulfonation of mixtures containing alkyl glycosides characterized in that a homogeneous mixture - liquid at temperatures of 25 to 80 °C - of

a) an alkyl glycoside corresponding to general formula **(I)**

**R-O-(G)$_p$**     **(I)**

in which G stands for a glycose unit derived from a sugar containing 5 or 6 carbon atoms, the index p represents the degree of oligomerization and is a number of 1 to 10 and R is a $C_{6-22}$ alkyl radical, and

b) a compound selected from the group consisting of
- b[1)] fatty alcohols containing 6 to 22 carbon atoms,
- b[2)] adducts of, on average, 0.1 to 10 mol ethylene oxide with fatty alcohols containing 6 to 22 carbon atoms,
- b[3)] saturated and unsaturated fatty acids containing 6 to 22 carbon atoms and
- b[4)] esters of saturated and unsaturated fatty acids containing 6 to 22 carbon atoms with alcohols containing 1 to 5 carbon atoms

is sulfonated and subsequently neutralized with aqueous bases.

**2.** A process as claimed in claim 1, characterized in that alkyl glucosides are used in which the glycose unit G in general formula **(I)** is derived from aldoses or ketoses, particularly glucose, the average degree of oligomerization p is a number of 1.1 to 3.0 and, more particularly, 1.1 to 1.4 and R is an alkyl radical containing 12 to 18 carbon atoms.

**3.** A process as claimed in claim 1 or 2, characterized in that fatty alcohols containing 12 to 18 carbon atoms are used as component b) of the mixture.

**4.** A process as claimed in claim 3, characterized in that a technical coconut oil alcohol is used as component b) of the mixture.

**5.** A process as claimed in claim 1 or 2, characterized in that adducts of, on average, 1 to 8 mol ethylene oxide with fatty alcohols containing 6 to 22 carbon atoms are used as component b) of the mixture.

9

6. A process as claimed in claim 5, characterized in that the hydrotalcite-catalyzed addition product of, on average, 5 mol ethylene oxide with a technical $C_{12/14}$ coconut oil alcohol cut is used as component b) of the mixture.

7. A process as claimed in claim 1 or 2, characterized in that saturated or unsaturated fatty acids containing 12 to 20 carbon atoms and 0, 1, 2 or 3 double bonds are used as component b) of the mixture.

8. A process as claimed in claim 7, characterized in that technical oleic acid containing less than 10% by weight saturated secondary products is used as component b) of the mixture.

9. A process as claimed in claim 1 or 2, characterized in that esters of fatty acids containing 12 to 22 carbon atoms and 0, 1, 2 or 3 double bonds with aliphatic alcohols containing 1 to 5 carbon atoms are used as component b) of the mixture.

10. A process as claimed in claim 9, characterized in that methyl esters of technical coconut oil and tallow fatty alcohols are used as component b) of the mixture.

11. A process as claimed in at least one of claims 1 to 10, characterized in that mixtures in which the ratio of component a) to component b) is 60:40 to 5:95% by weight are used.

12. A process as claimed in claim 11, characterized in that the ratio of component a) to component b) in the mixture is 50:50 to 15:85% by weight.

13. A process as claimed in at least one of claims 1 to 12, characterized in that mixtures of $C_{12/14}$ coconut oil alkyl glucosides and a technical $C_{12/14}$ coconut oil fatty alcohol cut in a ratio by weight of 20:80 to 30:70 are used.

14. A process as claimed in at least one of claims 1 to 13, characterized in that the sulfonation of the mixtures of a) with b) is carried out with sulfuric acid, oleum, chlorosulfonic acid or amidosulfonic acid, but especially with gaseous sulfur trioxide in admixture with an inert gas.

15. A process as claimed in at least one of claims 1 to 14, characterized in that the sulfonation reaction is carried out with a molar ratio of starting mixture to gaseous sulfur trioxide of 1:0.9 to 1:1.6.

16. A process as claimed in at least one of claims 1 to 15, characterized in that the sulfonation is carried out at temperatures of 25 to 80°C.

17. A process as claimed in at least one of claims 1 to 16, characterized in that the acidic sulfonation products are neutralized with aqueous bases selected from the group of alkali metal hydroxides, alkaline earth metal oxides and hydroxides, ammonia, mono-, di- and tri- $C_{2-4}$ alkanolamines and primary, secondary and tertiary $C_{1-4}$ alkylamines.

**Revendications**

1. Procédé de sulfonation de mélanges contenant des alcoylglycosides, caractérisé en ce que l'on sulfonate un mélange homogène, liquide à des températures comprises entre 25 et 80°C, formé de :
   a) un alcoylglycoside de formule générale I

   R-O-(G)$_p$

   dans laquelle G représente un symbole pour une unité de glycose qui dérive d'un sucre ayant 5 ou 6 atomes de carbone et dont le degré d'oligomérisation p représente un nombre entre 1 et 10 et R représente un radical alcoyle ayant de 6 à 22 atomes de carbone et
   b) un composé choisi dans le groupe formé de :
      b$^1$) des alcools gras ayant de 6 à 22 atomes de carbone.
      b$^2$) les produits d'addition d'oxyde d'éthylène en moyenne de 0,1 à 10 mol, sur des alcools gras ayant de 6 à 22 atomes de carbone.

10

b³)     des acides gras saturés et non saturés ayant de 6 à 22 atomes de carbone et,

b⁴)     des esters d'acides gras saturés et non saturés, ayant de 6 à 22 atomes de carbone avec des alcools ayant de 1 à 5 atomes de carbone.

et qu'ensuite on neutralise avec des bases aqueuses.

2.  Procédé selon la revendication 1, caractérisé en ce qu'on utilise des alcoylglucosides pour lesquels l'unité de glycose G dans la formule générale I dérive d'aldoses ou de cétoses et en ce que le degré d'oligomérisation. moyen p représente un nombre allant de 1,1 à 3,0, en particulier de 1,1 à 1,4 et R représente un radical alcoyle ayant de 12 à 18 atomes de carbone.

3.  Procédé selon l'une des revendications 1 et 2, caractérisé en ce que l'on utilise comme composant du mélange b) des alcools gras ayant de 12 à 18 atomes de carbone.

4.  Procédé selon la revendication 3, caractérisé en ce que comme composent du mélange b) on utilise un alcool de coco industriel.

5.  Procédé selon l'une des revendications 1 et 2, caractérisé en ce que comme composant du mélange b) on utilise des produits d'addition de 1 à 8 moi d'oxyde d'éthylène en moyenne sur des alcools gras ayant de 6 à 22 atomes de carbone.

6.  Procédé selon la revendication 5, caractérisé en ce que l'on utilise comme composant du mélange b) le produit d'addition catalysé par l'hydrotalcite, de 5 mol d'oxyde d'éthylène en moyenne sur une coupe d'alcool de coco en $C_{12}$-$C_{14}$ industrielle.

7.  Procédé selon l'une des revendications 1 et 2, caractérisé en ce que l'on utilise comme composant du mélange b) des acides gras saturés et non saturés ayant de 12 à 20 atomes de carbone et 0,1, 2 ou 3 doubles liaisons.

8.  Procédé selon la revendication 7, caractérisé en ce que l'on utilise comme composant du mélange b) un acide oléique industriel ayant un pourcentage inférieur à 10 % en poids de sous-produits saturés.

9.  Procédé selon l'une des revendications 1 et 2, caractérisé en ce que l'on utilise comme composant du mélange b) un ester d'acides gras ayant de 12 à 22 atomes de carbone et 0,1, 2 ou 3 doubles liaisons, avec des alcools aliphatiques ayant de 1 à 5 atomes de carbone.

10.  Procédé selon la revendication 9, caractérisé en ce que l'on utilise comme composant du mélange b) un ester méthylique d'acides gras industriels de coco et de suif.

11.  Procédé selon au moins une des revendications 1 à 10, caractérisé en ce que l'on utilise des mélanges, dans lesquels le rapport du mélange du composant a) au composent b) s'élève de 60:40 à 5:95 % en poids.

12.  Procédé selon la revendication 11, caractérisé en ce que l'on utilise des mélanges, dans lesquels le rapport du mélange du composant a) au composant b) s'élève de 50:50 à 15:85 % en poids.

13.  Procédé selon au moins une des revendications 1 à 12, caractérisé en ce que l'on utilise des mélanges d'(alcoyle de coco en C12/$C_{14}$) glucosides et d'une coupe industrielle d'alcool gras de coco en $C_{12}$/$C_{14}$ dans le rapport pondéral allant de 20:80 à 30:70.

14.  Procédé selon au moins l'une des revendications 1 à 13, caractérisé en ce que la sulfonation des mélanges de a) et de b) est effectuée avec l'acide sulfurique, l'oléum, l'acide chlorosulfonique ou l'acide amidosulfonique, en particulier cependant avec de l'anhydride sulfurique gazeux en mélange avec un gaz inerte.

15.  Procédé selon au moins une des revendications 1 à 14, caractérisé en ce que l'on effectue la sulfonation avec un rapport d'utilisation molaire du mélange de départ à l'anhydride sulfurique gazeux allant de 1.0,9 à 1:1,6.

16. Procédé selon au moins une des revendications 1 à 15, caractérisé en ce que l'on effectue la sulfonation à des températures allant de 25 à 80°C.

17. Procédé selon au moins une des revendications 1 à 16, caractérisé en ce que l'on effectue la neutralisation des produits sulfonés acides avec des bases aqueuses choisies dans le groupe formé des hydroxydes de métal alcalin, des oxydes et hydroxydes de métal alcalino-terreux, de l'ammoniac, des mono-, di- et tri(alcanol en $C_2$-$C_4$) amines ainsi que des (alcoyle en $C_1$ à $C_4$) amines, secondaires et tertiaires.